# EUROPÄISCHE PATENTSCHRIFT

(11) **EP 2 739 718 B1**
(45) Veröffentlichungstag und Bekanntmachung des Hinweises auf die Patenterteilung: **18.05.2016**
(21) Anmeldenummer: 12745784.4
(22) Anmeldetag: 02.08.2012
(51) Int. Cl.: C12M 1/107

(54) **FERMENTERSYSTEM SOWIE VERFAHREN ZUR KONTINUIERLICHEN FERMENTATION**
FERMENTER SYSTEM AND METHOD FOR CONTINUOUS FERMENTATION
SYSTÈME FERMENTEUR ET PROCÉDÉ DE FERMENTATION EN CONTINU

(30) Priorität: 04.08.2011 DE 102011109430
(43) Veröffentlichungstag der Anmeldung: 11.06.2014
(73) Patentinhaber: Friedmann, Hans, 85296 Rohrbach (DE)
(72) Erfinder: Friedmann, Hans, 85296 Rohrbach (DE)
(74) Vertreter: Rösler, Uwe
(86) Internationale Anmeldenummer: PCT/EP2012/003333
(87) Internationale Veröffentlichungsnummer: WO 2013/017291

(56) Entgegenhaltungen:
- WO-A2-2010/028643

## Beschreibung

### Technisches Gebiet

Die Erfindung bezieht sich auf ein Fermentersystem zur kontinuierlichen Fermentation von landwirtschaftlichen Kulturpflanzen, insbesondere von Rüben oder Zuckerrüben oder von aus landwirtschaftlichen Kulturpflanzen gewonnenen Zwischenprodukten, im weiteren als "Ausgangsmaterial" bezeichnet, mit einem Vorratslager zur Bevorratung des Ausgangsmaterials, einer Zuführeinrichtung zur Beschickung eines Fermenters mit dem bevorrateten Ausgangsmaterial aus dem Vorratslager, sowie einem Ausbringlager, in das mittels einer Verbindungseinrichtung fermentiertes Restmaterial aus dem Fermenter einbringbar ist. Ferner wird ein Verfahren zur kontinuierlichen Fermentation von landwirtschaftlichen Kulturpflanzen, insbesondere von Rüben oder Zuckerrüben oder von aus landwirtschaftlichen Kulturpflanzen gewonnenen Zwischenprodukten beschrieben.

### Stand der Technik

Fermenteranlagen bzw. -systeme der vorstehenden Gattung dienen u. a. zur Herstellung technisch nutzbarer Biogase, die beispielsweise zur Energiegewinnung dem Verbrennungsprozess eines Biomassekraftwerkes oder einer Gas-Brennstoffzelle zugeführt werden. Seit Beginn der technischen Nutzbarmachung von aus nachwachsenden Energierohstoffen im Wege der Vergärung und/oder von Fermentierungsprozessen gewonnenen Biogasen besteht ein vornehmliches Entwicklungsziel darin, sämtliche hierzu erforderlichen Anlagenkomponenten sowie die zur Energiegewinnung erforderlichen Einzelprozesse sowohl unter ökonomischen als auch ökologischen Aspekten zu optimieren.

Bspw. wird in der deutschen Patentschrift DE 10 2007 011 783 B4 zur Reduzierung umweltbelastender logistischer Transportwege ein Verfahren vorgeschlagen, bei dem zum Betrieb einer Biomasse-Energieanlage die räumliche Nähe eines Biomassekraftwerkes zur Anbau- und. Aufwuchsfläche von Biomassepflanzen gefordert wird, auf der die Pflanzen partiellenweise zeitversetzt geerntet und direkt zur benachbart angeordneten Biomasse-Energieanlage transportiert und dort verwertet werden. Um einen ganzjährigen kontinuierlichen Betrieb der Biomasse-Energieanlage sicherzustellen, sieht das bekannte Biomassekraftwerk wenigstens einen hybridischen Verbrennungsofen vor, der sowohl die Verfeuerung von direkt von der benachbarten Aufwuchsfläche geernteten und geheckselten Biomasse erlaubt, als auch die Verbrennung von aus der Biomasse zuvor gewonnenem Biogas ermöglicht. So können sowohl die während der sommerlichen Trockenphase geerntete trockene Biomasse im Wege einer direkten Verbrennung sowie auch die während der winterlichen Nassphase geerntete feuchte Biomasse im Wege einer Fermentierung und dabei erfolgenden Biogaserzeugung zur Energiegewinnung im Rahmen des Biomassekraftwerkes genutzt werden.

Soll hingegen der ganzjährige kontinuierliche Betrieb eines Biomassekraftwerkes lediglich auf Basis von Biogas erfolgen, so bedarf es einer pausenlosen Bereitstellung bzw. Erzeugung von Biogas. Dies wiederum setzt voraus, dass die von den landwirtschaftlichen Ertragsflächen geerntete Biomasse in einem Umfang zu bevorraten ist, so dass ein Biogaserzeugender Fermentationsprozess kontinuierlich aufrechterhalten werden kann. Geht man bei der dem Fermentationsprozess kontinuierlich zuzuführenden Biomasse von einer landwirtschaftlichen Kulturpflanze aus, die nach entsprechender Aufbereitung dem Fermentationsprozess zur Herstellung von Biogas zugeführt wird, so bedarf es durchaus großer Lagerkapazitäten, um den Jahresbedarf an Biogas einer kontinuierlich betriebenen Biomassekraftwerksanlage sicherzustellen. Hinzukommt, dass das während der Fermentation anfallende, technisch zumindest nicht unmittelbar nutzbare fermentierte Restmaterial in Auffanglager zwischen zu lagern ist, bis eine Ausbringung bspw. zur Düngung der Felder möglich ist. Dies ist zumeist im Frühjahr sowie Herbst der Fall.

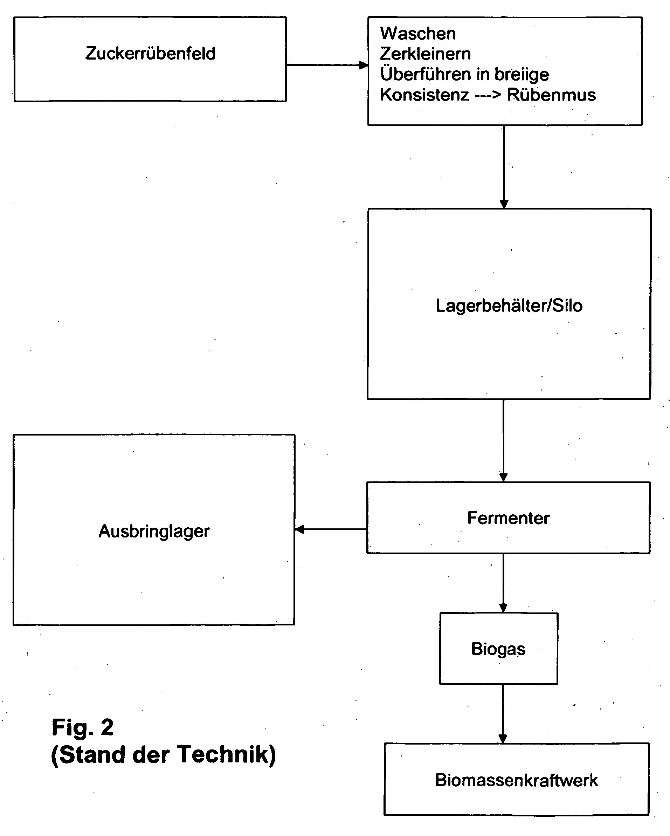

Unter Bezugnahme auf das in Figur 2 dargestellte Prozessdiagramm sei die bisherige Ernte- und Lagerungspraxis von Rüben, insbesondere Zuckerrüben als zu bevorratende Biomasse zur Erzeugung von technisch nutzbarem Biogas im Rahmen eines Fermentierungsprozesses illustriert. Zuckerrüben sind Feldfrüchte, die über einen hohen Flüssigkeits- und geringen Faseranteil verfügen und einmal im Jahr ab Mitte September bis Mitte November geerntet werden, wobei eine spätere Ernte bei guter Witterung zu einem erhöhten Zuckergehalt aufgrund der längeren Vegetationszeit führt, der sich wiederum vorteilhaft bei der Gärung bzw. Fermentation in Bezug auf eine verbesserte Biogasgewinnung auswirkt. Nach dem Ernteschritt werden die Zuckerrüben gewaschen, zerkleinert und einer Vorbehandlung unterzogen, bei der die zerkleinerten Zuckerrüben eine breiige Konsistenz in Form eines Zuckerrübenmus annehmen, das im Vergleich zu unbehandelten Rüben bzw. lediglich zerkleinerten Rüben ein geringeres Lagervolumen benötigt. Gleichwohl gilt es die Lagerbehälter derart groß zu dimensionieren, so dass genügend Aufnahmekapazität für Rübenmus vorhanden ist, dessen sukzessive Fermentierung innerhalb eines Fermenters Biogas in ausreichendem Maße zu erzeugen in der Lage ist, das einen ganzjährigen kontinuierlichen Betrieb eines Biomassekraftwerkes sicherstellt.

Ferner gilt es wenigstens zur zeitlichen Zwischenlagerung des im Rahmen des Fermentierungsprozesses technisch nicht nutzbaren fermentierten Restmaterials Speichervorkehrungen, im Weiteren als Ausbringlager bezeichnet, vorzusehen, die zu bestimmten Zeiten im Jahr entleert werden können, bspw. zu Zwecken der Düngung von Feldern. Typischerweise erfolgt die Ausbringung des Restmaterials auf die Felder im Frühjahr sowie zum Herbst. Dies jedoch setzt wiederum große Lagerkapazitäten voraus, um das fermentierte Restmaterial mit ausreichendem Volumenumfang zwischen zu lagern.

Aus der US 6 059 972 A ist eine Anlage zur anaeroben Biokonversion von organischen Abfällen, z.B. aus Restaurantküchen, bekannt, bei der Biogas entsteht. Die Abfälle werden zerkleinert und verbleiben zunächst für einen Tag in einem, im Biokonversionstank angeordneten zweiten Bereich, wo sie vorerhitzt werden und eine erste Gasbildungsphase beginnt. Anschließend gelangen die vorbehandelten Abfälle portionsweise in den Tank, wobei durch eine aufwändige Rezirkulationsanlage die Bedingungen für eine kontinuierliche Biomethanisierung kontrolliert werden.

In der Druckschrift DE 10 2008 015 609 A1 ist ein Verfahren zur Herstellung von Biogas in einem mehrstufigen Prozess bekannt, bei dem der Hydrolysevorgang räumlich getrennt vom Methanbildungsprozess erfolgt. Das Ausgangsmaterial wird zunächst in zwei separaten Behältern, die zeitlich versetzt befüllt werden, hydrolisiert und anschließend in den Fermenter zur Herstellung von Biogas überführt. Zum Aufmischen der "neuen" Biomasse, die in eine der Hydrolyseeinheiten gegeben wird, wird eine Flüssigfraktion, zurück zu den Hydrolyseeinheiten geführt.

In der Druckschrift EP 0 795 022 B1 sind eine Verarbeitungsanlage für festen Hausmüll sowie ein Prozess für die kommerzielle Herstellung von Milchsäure offenbart. Aus dem Hausmüll wird über diverse Verfahrensschritte eine Zucker enthaltende Lösung erhalten, die mittels Milchsäurebakterien fermentiert wird, um Milchsäure zu enthalten. In erstem Behälter befindet sich die zuckerhaltige Lösung, wohingegen der Behälter eine Zucker/Ammonium/Bakterien-Mischung enthält.

Die DE 10 2007 063 091 A1 beschreibt einen Hybridfermenter zur Erzeugung von methanreichem Biogas, mit einem Wasserstoff- und einem Methanfermenter. Der Wasserstofffermenter wird mit Substrat aus dem Methanfermenter oder über ein eigenständiges Aufbereitungs- und Zuführungssystem mit speziellem Substrat versorgt. Ebenso werden die Rohstoffe auch vor der Zuführung in den Methanfermenter bearbeitet.

Aus WO 2010/028643 A2 ist ein Fermentersystem mit einem Vorratslager in Form von mehreren Lagerbehältern (Vorlagebehälter) sowie ein Ausbringlager in Form von mehreren Lagerbehältern bekannt. In den Vorlagebehältern können auch Rezirkulate rückgeführt werden, bevor sie einem Fermenter zugeführt werden.

### Darstellung der Erfindung

Der Erfindung liegt die Aufgabe zugrunde, ein Fermentersystem zur kontinuierlichen Fermentation von landwirtschaftlichen Kulturpflanzen, insbesondere von Rüben oder Zuckerrüben oder von aus landwirtschaftlichen Kulturpflanzen gewonnenen Zwischenprodukten, im Weiteren als Ausgangsmaterial bezeichnet, mit einem Vorratslager zur Bevorratung des Ausgangsmaterials, einer Zuführeinrichtung zur Beschickung eines Fermenters mit dem bevorrateten Ausgangsmaterial aus dem Vorratslager, sowie einem Ausbringlager, in das mittels einer Verbindungseinrichtung fermentiertes Restmaterial aus dem Fermenter einbringbar ist, derart weiterzubilden, dass trotz der Forderung nach einer kontinuierlichen Fermentation und einer damit verbundenen kontinuierlichen, d.h. möglichst ganzjährigen Herstellung von Biogas das für die Bevorratung des vorzugsweise in Form von Rüben oder Zuckerrüben vorliegenden Ausgangsmaterials erforderliche Lagervolumen deutlich reduziert werden soll.

Insbesondere gilt es das für einen kontinuierlichen Ganzjahresbetrieb eines Fermentersystems zu bevorratende Biomassevolumen, vorzugsweise in Form von Zuckerrüben, möglichst gering zu halten, um sowohl den Platzbedarf als auch die Kosten für die Bevorratung von Zuckerrüben zu reduzieren. Selbstverständlich soll sich das Fermentersystem auch für die Verarbeitung sämtlicher für die Fermentation geeigneter Biomasse eignen.

Ferner gilt es ein diesbezügliches Verfahren anzugeben, das sowohl ökonomische als auch ökologische Vorteile gegenüber der bisherigen Lagerungs- bzw. Bevorratungspraxis bietet.

Die Lösung der der Erfindung zugrunde liegenden Aufgabe ist im Anspruch 1 angegeben. Gegenstand des Anspruches 7 ist ein lösungsgemäßes Verfahren. Den Erfindungsgedanken mit vorteilhafter Weise weiterbildende Merkmale sind Gegenstand der Unteransprüche sowie der weiteren Beschreibung insbesondere unter Bezugnahme auf die Ausführungsbeispiele zu entnehmen.

Die der Erfindung zugrunde liegende Idee sieht eine kombinierte Nutzung von wenigstens drei getrennten Lagerbehältern sowohl für die Bevorratung von Ausgangsmaterial, vorzugsweise in Form von vorbehandelten Rüben, insbesondere Zuckerrüben, die in breiiger Konsistenz als Rübenmus vorliegen, als auch zur Zwischenlagerung von fermentiertem Restmaterial vor, das typischerweise zum Ausbringen auf die Felder zu Düngungszwecken gesammelt wird. So zeichnet sich ein lösungsgemäß ausgebildetes Fermentersystem nach den Merkmalen des Oberbegriffes des Anspruches 1 dadurch aus, dass das Vorratslager und das Ausbringlager in Form von wenigstens drei Lagerbehältern, ein erster, ein zweiter und dritter Lagerbehälter, ausgebildet sind, von denen der erste und zweite Lagerbehälter zumindest über eine Zuführeinrichtung, über die das in den Lagerbehälter lagernde Ausgangsmaterial in den Fermenter überführbar ist, sowie der zweite und dritte Lagerbehälter zumindest über eine Verbindungseinrichtung, über die das in dem Fermenter entstehende technisch nicht nutzbare fermentierte Restmaterial ausleitbar ist, mit dem Fermenter verbunden sind. Außerdem verfügen der zweite und dritte Lagerbehälter (L2, L3) jeweils über eine Ablassleitung (AB), über die das in den jeweiligen Lagerbehältern (L2, L3) zwischengelagerte fermentierte Restmaterial (R) entleerbar ist.

Um die aus wenigstens drei Lagerbehältern bestehenden Vorrats- und Ausbringlager jeweils in chronologischer und aufeinander abgestimmter Abfolge sowohl mit dem zu fermentierenden Ausgangsmaterial, vorzugsweise in Form von Zuckerrübenmus, sowie auch nach entsprechender Fermentation des Ausgangsmaterials innerhalb des Fermenters mit dem fermentieren Restmaterial zu befüllen, ist eine Steuereinrichtung vorgesehen, die einen aus dem Ausgangsmaterial oder dem fermentierten Restmaterial bestehenden Materialfluss zwischen den Lagerbehältern und dem Fermenter längs der jeweils vorgesehenen Zuführ- und Verbindungseinrichtungen, koordiniert bzw. steuert.

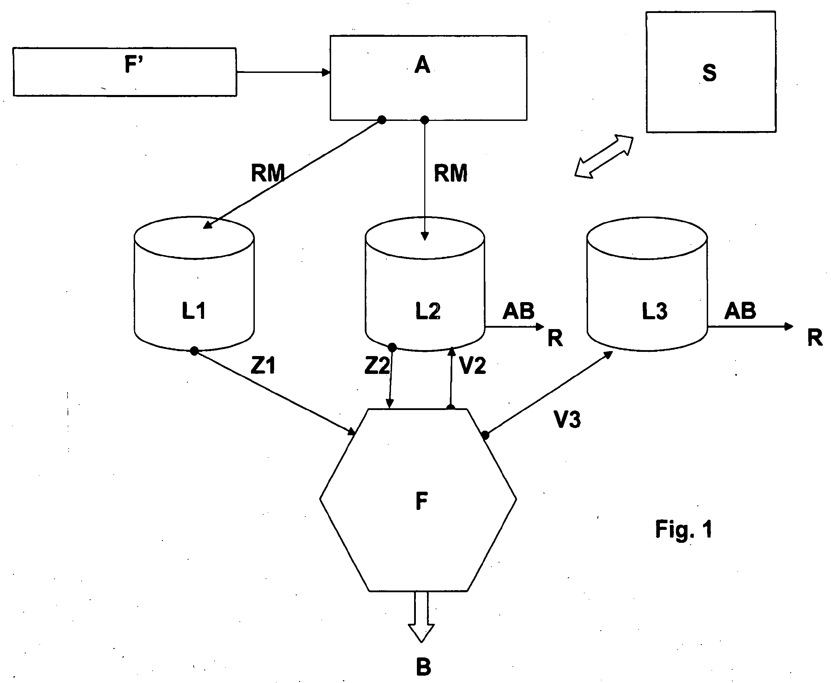

Das lösungsgemäße Fermentersystem sei zur besseren Illustration und Erläuterung anhand des in Figur 1 entnehmbaren Schemas im Weiteren beschrieben, das jedoch den allgemeinen Erfindungsgedanken nicht beschränken soll. So ist es grundsätzlich denkbar mehr als drei Lagerbehälter vorzusehen oder anstelle von Zuckerrüben als biologisches Ausgangsmaterial alternative nachwachsende Energierohstoffe einzusetzen, so bspw. vorbereitete biologische Substrate, wie Schlempe etc..

In Figur 1 sind jeweils der erste, zweite und dritte Lagerbehälter mit den Abkürzungen L1, L2, L3 gekennzeichnet. Der erste Lagerbehälter L1 ist mit dem Fermenter F über eine Zuführeinrichtung Z1 verbunden, über die das im Lagerbehälter L1 bevorratete Ausgangsmaterial dosiert in den Fermenter F überführbar ist. Gleichsam ist der zweite Lagerbehälter L2 mit einer entsprechenden Zuführeinrichtung Z2 mit dem Fermenter F, ebenfalls zur dosierten Zuführung von zu fermentierendem Ausgangsmaterial in den Fermenter F verbunden. Zugleich ist der Fermenter F mit dem zweiten Lagerbehälter L2 über eine Verbindungseinrichtung V2 verbunden, über die der zweite Lagerbehälter L2 bedarfsweise mit fermentiertem Restmaterial befüllt werden kann. Letztlich ist der dritte Lagerbehälter L3 über eine Verbindungseinrichtung V3 mit dem Fermenter F zur Befüllung des dritten Lagerbehälters L3 mit fermentiertem Restmaterial verbunden.

Der im Rahmen des Fermenters F stattfindende Fermentationsprozess erzeugt technisch nutzbares Biogas B, das zur Energiegewinnung einem nicht weiter dargestellten Biomassekraftwerk zugeleitet wird.
Der zweite und dritte Lagerbehälter L2, L3 verfügt jeweils zusätzlich über eine Ablassleitung AB, über die das in den jeweiligen Lagerbehältern L2, L3 zwischengelagerte fermentierte Restmaterial FR entleert werden kann.

Eine Steuereinrichtung S, sorgt für einen geordneten Materialfluss zwischen den einzelnen Lagerbehältern L1, L2, L3 und dem Fermenter F, bspw. durch Ansteuerung von nicht weiter in der Figur 1 längs der einzelnen Zuführ- und Verbindungseinrichtungen Z1, Z2, V2, V3, AB vorgesehenen Steuerventilen.

Im Weiteren sei davon ausgegangen, dass in einer Ausgangssituation alle drei Lagerbehälter L1, L2 und L3 entleert sind. Diese Ausgangssituation herrscht zur

Erntezeit vor, die im Falle von Zuckerrüben als Ausgangsmaterial auf das Jahresende in die Monate Oktober/November fällt.

Die auf einem Feldareal F' geernteten Zuckerrüben werden in einer Aufbereitungseinheit A gewaschen, zerkleinert und zu einem Rübenmus verarbeitet, dessen Einlagerung das geringste Maß an Lagervolumen fordert. In der vorstehend zitierten Ausgangssituation werden die Lagerbehälter L1 und L2 mit entsprechendem Rübenmus RM vorzugsweise vollständig befüllt. Der Lagerbehälter L3 verbleibt zunächst leer und dient im Weiteren als Auffang- und Zwischenlagerbehälter für das im Rahmen der Fermentation zurückbleibende, fermentierte Restmaterial. Die Lagerbehälter sind vorzugsweise aus Edelstahl gefertigt und mit einer Durchmischungseinrichung für die in den Lagerbehältern enthaltende breiige Konsistenz ausgestattet, um sedimentative Separierprozesse innerhalb des zu bevorratenden Rübenmus zu verhindern. Grundsätzlich gilt es während der Lagerung des Rübenmuses innerhalb der Lagerbehälter L1 und L2 dafür zu sorgen, dass das Rübenmus biologisch stabilisiert wird, z.B. durch eine Silierung, im Wege einer Milch- oder Essigsäuregärung.

In einem ersten Fermentationsschritt wird der Fermenter F durch dosierte Zugabe von Rübenmus aus dem zweiten Lagerbehälter L2 über die Zuführeinrichtung Z2 gespeist. Die dosierte Materialzuführung an Rübenmus erfolgt unter Maßgabe der innerhalb des Fermenters F erfolgenden Umsetzung von Rübenmus in Biogas B und in fermentiertes Restmaterial im Rahmen des Fermentationsprozesses. Die Materialzuführung von zu fermentierendem Rübenmus aus dem zweiten Lagerbehälter L2 erfolgt gesteuert über die Steuereinrichtung S unter Maßgabe einer möglichst kontinuierlichen Biogasproduktion, bei der ein kontinuierlicher Biogasstrom B aus dem Fermenter F abgeleitet werden kann, der nachfolgend zu einer kontinuierlichen Einspeisung als Brennstoff in einem Verbrennungsprozess eines Biomassekraftwerkes genutzt werden kann. Auch die Abführung des fermentierten Restmaterials R über die Verbindungseinrichtung V3 in den dritten Lagerbehälter L3 efolgt kontinuierlich, so dass sich innerhalb des Fermenters F ein bleibendes Fermentationsmilieu einstellen kann. Die Zuführung von Rübenmus aus dem zweiten Lagerbehälter L2 erfolgt solange, bis der zweite Lagerbehälter vollständig entleert ist.

Um die Kontinuität des innerhalb des Fermenters F stattfindenden Fermentationsprozesses aufrechtzuerhalten, schließt die Steuereinrichtung S eine weitere Stoffzuführung über die Zuführeinrichtung Z2 zwischen dem zweiten Lagerbehälter L2 und dem Fermenter F und öffnet zeitgleich die Zuführeinrichtung Z1 zwischen dem ersten Lagerbehälter L1 und dem Fermenter F, so dass nun Rübenmus aus dem ersten Lagerbehälter L1 in dosierter Form in den Fermenter F gelangen kann.

Sofern der dritte Lagerbehälter L3 nicht bereits vollständig mit fermentiertem Restmaterial R gefüllt ist, so gelangt auch weiterhin das fermentierte Restmaterial, das im Rahmen des Fermentationsprozesses kontinuierlich gebildet wird, in den dritten Lagerbehälter L3. Für den Fall, dass der dritte Lagerbehälter L3 vollständig gefüllt ist, so wird die Verbindungseinrichtung V2 zwischen Fermenter F und dem zweiten Lagerbehälter, der nun entleert ist, geöffnet, wohingegen die Verbindungseinrichtung V3 durch die Steuereinrichtung S geschlossen wird, um eine weitere Befüllung des Lagerbehälters L3 zu verhindern.

Der in dieser Phase innerhalb des Fermenters F stattfindende Fermentationsprozess wird durch die dosierte Zugabe von Rübenmus aus dem Lagerbehälter L1 und die dosierte Abgabe von fermentiertem Restmaterial R in den Lagerbehälter L2 sowie die Abgabe von technisch nutzbaren Biogas B bestimmt.

Die Dimensionierung der einzelnen Lagerbehälter L1, L2 und L3 ist nicht notwendigerweise mit jeweils identischem Lagervolumina auszuführen, bspw. ist es sicherlich vorteilhaft das Lagervolumen der Lagerbehälter L1 und L2 größer als das Lagervolumen des Lagerbehälters L3 auszuführen, zumal das spezifische Lagervolumen des fermentierten Restmaterials R kleiner ist als das für das bevorratete Rübenmus.

Beginnt sich auch der erste Lagerbehälter L1 zu entleeren, so bietet es sich an, um den Fermentationsprozess innerhalb des Fermenters F nicht zu unterbrechen, jeweils den zweiten und dritten Lagerbehälter L2, L3 über die Ableitungen AB zu entleeren, und das fermentierte. Restmaterial zu Düngungszwecken auf das Feldareal auszutragen. Nachfolgend können der erste und zweite Lagerbehälter sukzessive mit weiterem Rübenmus befüllt werden, noch bevor eine vollständige Entleerung des ersten Lagerbehälters erfolgt, um den eingangs erläuterten Ausgangszustand wieder zu erhalten.

In einem bevorzugten Ausführungsbeispiel sind die Aufnahmekapazitäten der einzelnen Lagerbehälter L1, L2 und L3 derart dimensioniert, dass die vorstehend erläuterte vollständige Entleerung der Lagerbehälter L1, L2, L3 zweimal im Jahr stattfindet, nämlich im Frühjahr und Herbst zur Erntezeit. Sind die Lagerbehälter L1, L2, L3 im Frühjahr, vorzugsweise im März, entleert, so kann erneutes Rübenmus in die Lagerbehälter L1 und L2 aufgenommen werden, zu einer Zeit, in der die winterlichen Temperaturen im Durchschnitt noch genügend tief sind, so dass eine freie Lagerung von im Vorjahr geernteten Zuckerrüben an der Atmosphäre im Bereich des Feldareals F ohne Beeinträchtigung der Zuckerrübenqualität möglich ist. Mit der Lagernutzung am freien Feld während der Wintermonate ist es bei der Ernte nicht erforderlich das gesamte Erntevolumen an Zuckerrüben in Rübenmus zu verarbeiten und in großvolumige Lagerstätten unterzubringen. Vielmehr kann etwa die Hälfte des jährlichen Ernteertrages an Zuckerrüben im Feldareal in unbehandelter Form bei winterlichen Temperaturen zwischengelagert werden. Die im Feldareal seit der Ernte des Vorjahres geernteten und zwischengelagerten Zuckerrüben können nun im Folgejahr im März, d. h. vor einer Jahreszeit mit signifikant ansteigenden Temperaturen, der Aufbereitungseinheit A zu Zwecken der Reinigung, Zerkleinerung und Überführung in Rübenmus RM zugeführt werden. Auch ist es möglich eben zu dieser Jahreszeit im Frühjahr das bislang im zweiten und dritten Lagerbehälter gesammelte fermentierte Restmaterial auf dem Feldareal F problemlos auszutragen.

Nach entsprechender erneuten Befüllung des ersten und zweiten Lagerbehälters L1 und L2 und Entleerung des dritten Lagerbehälters L3 erfolgt der Betrieb des Fermenters F in der gleichen Weise wie vorstehend erläutert während des Winterzeitraumes. In gleicher Weise wie im Frühjahr erfolgt eine Entleerung aller drei Lagerbehälter L1, L2, L3 zur Erntezeit der Zuckerrüben Ende des Jahres im Zeitraum September/Oktober.

Das lösungsgemäße Fermentersystem nutzt demzufolge bei geeigneter Dimensionierung der Lagerbehälter L1, L2 und L3 die vorteilhafte und kostenlose Lagerung von Zuckerrüben in dem Zeitraum zwischen Oktober/November und März im Bereich des Feldareals F ohne weitere kostenintensive Vorkehrungen und ermöglicht durch kombinierte Nutzung der Lägerbehälter L1, L2 und L3 sowohl zu Lagerzwecken von Rübenmus als auch zu Zwecken der Lagerung des fermentierten Restmaterials einen kontinuierlichen Betrieb eines Fermenters mit erheblich reduziertem Lagerumfang und damit verbundenen geringeren Lagerkosten. Hinzukommt, dass durch eine zeitliche Entzerrung für die Befüllung der Lagerbehälter die Leistungsanforderungen an die Aufbereitungseinheit sehr viel geringer sind als im herkömmlichen Falle, bei dem eine Verarbeitung der vollständigen geernteten Feldfrüchte im Herbst zu erfolgen hat. Durch das lösungsgemäße Lagerkonzept reichen weniger leistungsstarke Aufbereitungseinheiten, die erheblich günstiger in der Anschaffung sowie im Betrieb sind, aus.

Um die Lebensdauer der einzelnen Lagerbehälter sowie den Kontaminationsschutz des in den Lagerbehältern zu bevorratenden Rübenmuses gegenüber äußeren Schadeinwirkungen zu verbessern, bestehen die Lagerbehälter aus einem korrosionsbeständigen Material. Ferner sind wenigstens jene Lagerbehälter, L1, L2, in die das zu bevorratende Rübenmus einbringbar ist, mit einer Durchmischungseinrichtung zur Begegnung sedimentativer Separierprozesse ausgestattet, um auf diese Weise für einen kontinuierlichen, unterbrechungsfreien Zufluss des Rübenmuses mit gleich bleibender Konsistenz in den Fermenter F zu sorgen. Typischerweise sieht eine derartige Durchmischungseinrichung ein innerhalb des jeweiligen Lagerbehälters angebrachtes Rührwerk vor oder ist mit einer Umwälzpumpe ausgestattet. Desweiteren kann zur Beeinflussung der breiigen Konsistenz des innerhalb des jeweiligen Lagerbehälters eingebrachten Rübenmuses eine Eindampf- und/oder Dehydrationseinrichtung vorgesehen sein, durch die der Flüssiganteil des in breiiger Konsistenz vorliegenden Rübenmuses reduziert werden kann. Selbstverständlich ist es möglich die breiige Konsistenz bereits innerhalb der vorstehend erläuterten Aufbereitungseinheit A entsprechend zu konditionieren.

Ferner gilt es die Lagerung des Rübenmuses zumindest innerhalb des ersten und zweiten Lagerbehälters L1 und L2 derart vorzusehen, dass die Lagerung unter Luftabschluss erfolgt, um auf diese Weise anaerobe Gärungsprozesse wie Milchsäuregärung oder Essigsäuregärung o. ä. zu fördern.

### Bezugszeichenliste

- F': Feldareal
- A: Aufbereitungseinheit
- S: Steuerungseinrichtung
- L1, L2, L3: Lagerbehälter
- RM: Rübenmus
- Z1, Z2: Zuführeinrichtung
- V2, V3: Verbindungseinrichtung
- F: Fermenter
- B: Biogas
- AB: Ableitung
- R: fermentiertes Restmaterial

## Patentansprüche

1. Fermentersystem zur kontinuierlichen Fermentation von landwirtschaftlichen Kulturpflanzen, insbesondere von Rüben oder Zuckerrüben oder von aus landwirtschaftlichen Kulturpflanzen gewonnenen Zwischenprodukten, im weiteren als Ausgangsmaterial bezeichnet, mit einem Vorratslager zur Bevorratung des Ausgangsmaterials, einer Zuführeinrichtung zur Beschickung eines Fermenters (F) mit dem bevorrateten Ausgangsmaterial aus dem Vorratslager, sowie einem Ausbringlager, in das mittels einer Verbindungseinrichtung fermentiertes Restmaterial (R) aus dem Fermenter (F) einbringbar ist,
**dadurch gekennzeichnet, dass** das Vorratslager und das Ausbringlager in Form von wenigstens drei Lagerbehältern (L1, L2, L3), ein erster, zweiter und dritter Lagerbehälter, ausgebildet sind, von denen der erste und zweite Lagerbehälter (L1, L2) zumindest über eine Zuführeinrichtung (Z1, Z2) mit dem Fermenter (F) verbunden ist, über die das in den Lagerbehältern (L1, L2) lagernde Ausgangsmaterial in den Fermenter (F) überführbar ist,
dass der zweite und dritte Lagerbehälter (L2, L3) zumindest über eine Verbindungseinrichtung (V2, V3) mit dem Fermenter (F) verbunden sind, über die das in dem Fermenter (F) entstehende technisch nicht nutzbare fermentierte Restmaterial (R) ausleitbar ist, und
dass der zweite und dritte Lagerbehälter (L2, L3) jeweils über eine Ablassleitung (AB) verfügen, über die das in den jeweiligen Lagerbehältern (L2, L3) zwischengelagerte fermentierte Restmaterial (R) entleerbar ist.

2. Fermentersystem nach Anspruch 1,
**dadurch gekennzeichnet, dass** eine Steuereinrichtung (S) vorgesehen ist, die einen aus dem Ausgangsmaterial oder fermentierten Restmaterial (R) bestehenden Materialfluss jeweils zwischen den Lagerbehältern (L1, L2, L3) und dem Fermenter (F) längs der jeweiligen Zuführ (Z1, Z2)- und Verbindungseinrichtung (V2, V3), ausgehend von mit Ausgangsmaterial gefüllten ersten und zweiten Lagerbehältern (L1, L2) sowie einem leeren dritten Lagerbehälter (L3) in der nachfolgenden Weise steuert:
a) Beschicken des Fermenters (F) mit Ausgangsmaterial aus dem zweiten Lagerbehälter (L2) und Befüllen des dritten Lagerbehälters (L3) mit fermentiertem Restmaterial (R) aus dem Fermenter (F) bis der zweite Lagerbehälter (L2) entleert und der dritte Lagerbehälter (L3) zumindest teilweise gefüllt ist,
b) Beschicken des Fermenters (F) mit Ausgangsmaterial aus dem ersten Lagerbehälter (L1) und Befüllen des zweiten Lagerbehälters (L2) mit fermentierten Restmaterial (R) aus dem Fermenter (F) bis der erste Lagerbehälter (L1) entleert ist und der zweite Lagerbehälter (L2) zumindest teilweise gefüllt ist und
c) Entleeren des zweiten und dritten Lagerbehälters (L2, L3), Befüllen des ersten und zweiten Lagerbehälters (L1, L2) mit Ausgangsmaterial und Wiederholen der Materialflussschritte a) und b).

3. Fermentersystem nach Anspruch 1 oder 2,
**dadurch gekennzeichnet, dass** der zweite und dritte Lagerbehälter (L2, L3) jeweils über ein Lagervolumen verfügt, das jeweils derart bemessen ist, dass sämtliches im zweiten Lagerbehälter (L2) bevorratete Ausgangsmaterial nach dem Fermentieren innerhalb des Fermenters (F) in Form von fermentierten Restmaterial (R) das Lagervolumen des dritten Lagerbehälters (L3) zumindest teilweise füllt, und dass der erste und zweite Lagerbehälter (L1, L2) jeweils über ein Lagervolumen verfügt, das jeweils derart bemessen ist, dass sämtliches im ersten Lagerbehälter (L1) bevorratete Ausgangsmaterial nach dem Fermentieren innerhalb des Fermenters (F) in Form von fermentierten Restmaterial (R) das Lagervolumen des zweiten Lagerbehälters (L2) zumindest teilweise füllt.

4. Fermentersystem nach einem der Ansprüche 1 bis 3,
**dadurch gekennzeichnet, dass** die Lagerbehälter (L1, L2, L3) aus einem korrosionsbeständigen Material bestehen.

5. Fermentersystem nach einem der Ansprüche 1 bis 4,
**dadurch gekennzeichnet, dass** wenigstens jene Lagerbehälter (L1, L2, in die das zu bevorratende Ausgangsmaterial durch eine Vorbehandlung in breiiger Konsistenz einbringbar ist, eine Durchmischungseinrichtung zur Begegnung sedimentativer Separierprozesse aufweisen.

6. Fermentersystem nach Anspruch 5,
**dadurch gekennzeichnet, dass** die Durchmischungseinrichtung ein innerhalb des Lagerbehälters (L1, L2) angebrachtes Rührwerk und /oder eine Umwälzpumpe vorsieht.

7. Verfahren zur kontinuierlichen Fermentation von landwirtschaftlichen Kulturpflanzen, insbesondere von Rüben, Zuckerrüben oder von aus landwirtschaftlichen Kulturpflanzen gewonnenen Zwischenprodukten, im weiteren als Ausgangsmaterial bezeichnet, bei dem das Ausgangsmaterial in wenigstens einem Lagerbehälter bevorratet wird zur sukzessiven Beschickung eines Fermenters (F), in dem das Ausgangsmaterial unter Bildung von Fermentationsprodukten in fermentiertes Restmaterial (R) umgewandelt wird, das in ein Ausbringlager überführt wird,
**dadurch gekennzeichnet, dass** die Bevorratung des Ausgangsmaterials in wenigstens zwei getrennten Lagerbehältern (L1, L2), einem ersten und einem zweiten Lagerbehälter, erfolgt, und
dass das Beschicken des Fermenters (F) mit dem Ausgangsmaterial nach den folgenden Schritten erfolgt:
a) Beschicken des Fermenters (F) mit Ausgangsmaterial aus dem zweiten Lagerbehälter (L2) und Befüllen eines dritten leeren Lagerbehälters (L3) mit technisch nicht nutzbarem fermentierten Restmaterial (R) aus dem Fermenter (F) bis der zweite Lagerbehälter (L2) vollständig entleert ist,
b) Beschicken des Fermenters (F) mit Ausgangsmaterial aus dem ersten Lagerbehälter (L1) und Befüllen des zweiten Lagerbehälters (L2) mit technisch nicht nutzbarem fermentierten Restmaterial (R) aus dem Fermenter (F) bis der erste Lagerbehälter (L1) entleert ist und
c) vollständiges Entleeren des zweiten und dritten Lagerbehälters (L2, L3), Befüllen des ersten und zweiten Lagerbehälters (L1, L2) mit Ausgangsmaterial und Wiederholen der Schritte a) und b).

8. Verfahren nach Anspruch 7,
**dadurch gekennzeichnet, dass** das Ausgangsmaterial vor dem Einbringen in die Lagerbehälter (L1, L2) vorbehandelt und in eine breiige, pumpfähige Konsistenz überführt wird, in der das Ausgangsmaterial dem Fermenter (F) zugeführt wird.

9. Verfahren nach Anspruch 8,
**dadurch gekennzeichnet, dass** die Vorbehandlung eine Reinigung und Zerkleinerung der das Ausgangsmaterial enthaltenden, geernteten Kulturpflanzen umfasst.

10. Verfahren nach einem der Ansprüche 7 bis 9,
**dadurch gekennzeichnet, dass** die Lagerung des Ausgangsmaterials in breiiger Konsistenz unter Luftabschluss erfolgt.

11. Verfahren nach einem der Ansprüche 7 bis 10,
**dadurch gekennzeichnet, dass** während der Lagerung des Ausgangsmaterials in breiiger Konsistenz eine Umwälzung des Ausgangsmaterials erfolgt zur Vermeidung sedimentativer Separationsprozesse.

12. Verfahren nach einem der Ansprüche 9 bis 11,
**dadurch gekennzeichnet, dass** nach der Vorbehandlung des Ausgangsmaterials und vor dem Zuführen des Ausgangsmaterials in den Fermenter (F) der Flüssiganteil des in breiiger Konsistenz vorliegenden Ausgangsmaterials durch Eindampfen und/oder mittels mechanischer Dehydrationstechnik reduziert wird.

13. Verwendung des Fementersystems nach einem der Ansprüche 1 bis 6 zur ganzjährigen, kontinuierlichen Herstellung von technisch nutzbaren Fermenterprodukten, insbesondere von Biogas.

14. Verwendung nach Anspruch 13,
**dadurch gekennzeichnet, dass** die technisch nutzbaren Fermenterprodukte Biogas ist.

## Claims

1. Fermenter system for the continuous fermentation of agricultural crop plants, in particular beet or sugar beet, or of intermediate products obtained from agricultural crop plants, hereafter designated as starting material, with a supply store for storing the starting material, a feed device for supplying a fermenter (F) with the stockpiled starting material from the supply store and with a discharge store, into which fermented residual material (R) can be channelled from the fermenter (F) by means of a connection device,
**characterized in that**
the supply store and the discharge store are implemented in the form of at least three storage containers (L1, L2, L3), a first, second and third storage container, of which the first and second storage containers (L1, L2) are connected to the fermenter (F) via at least one feed device (Z1, Z2), via which the starting material stored in the storage container can be channelled to the fermenter,
that the second and third storage containers (L2, L3) are connected to the fermenter (F) via at least one connection device (V2, V3), via which the fermented residual material (R) generated in the fermenter (F) but not technically usable can be discharged, and
that the second and third storage containers (L2, L3) each have an outlet line (AB), via which the fermented residual material (R) temporarily stored in the respective storage containers (L2, L3) can be emptied.

2. Fermenter system according to Claim 1,
**characterized in that**
a control device (S) is provided, which controls a material flow consisting of the starting material or fermented residual material (R) between each of the storage containers (L1, L2, L3) and the fermenter (F) along the respective feed device (Z1, Z2) and connection device (V2, V3), starting from first and second storage containers (L1, L2) filled with starting material and an empty third storage container (L3) in the following manner:
a) feeding of the fermenter with starting material from the second storage container and filling of the third storage container (L3) with fermented residual material (R) from the fermenter (F) until the second storage container (L2) is emptied and the third storage container (L3) is at least partially filled,
b) feeding of the fermenter (F) with starting material from the first storage container (L1) and filling of the second storage container (L2) with fermented residual material (R) from the fermenter (F) until the first storage container (L1) is emptied and the second storage container (L2) is at least partially filled, and
c) emptying of the second and third storage container (L2, L3), filling of the first and second storage containers (L1, L2) with starting material and repetition of the material flow steps (a) and (b).

3. Fermenter system according to Claim 1 or 2, **characterized in that** the second and third storage containers (L2, L3) each have a storage volume, each of which is dimensioned such that all starting material stockpiled in the second storage container (L2) in the form of fermented residual material (R) after the fermentation in the fermenter (F) at least partially fills the storage volume of the third storage container (L3), and
that the first and second storage containers (L1, L2) each have a storage volume, each of which is dimensioned such that all starting material stockpiled in the first storage container (L1) in the form of fermented residual material (R) after the fermentation in the fermenter (F) at least partially fills the storage capacity of the second storage container (L2).

4. Fermenter system according to any one of Claims 1 to 3,
**characterized in that** the storage containers (L1, L2, L3) are composed of a corrosion-resistant material.

5. Fermenter system according to any one of Claims 1 to 4,
**characterized in that** at least those storage containers (L1, L2), in which the starting material to be stockpiled can be brought into a pulpy consistency by a pre-treatment, have a mixing device for handling sediment-based separating processes.

6. Fermenter system according to Claim 5,
**characterized in that** the mixing device provides an agitator mounted within the storage container (L1, L2) and /or a circulation pump.

7. Method for the continuous fermentation of agricultural crop plants, in particular beet or sugar beet, or of intermediate products obtained from agricultural crop plants, hereafter designated as starting material, in which the starting material is stockpiled in at least one storage tank for the successive feeding of a fermenter (F), in which the starting material is converted by forming fermentation products into fermented residual material (R), which is channelled to a discharge store,
**characterized in that** the starting material is stockpiled in at least two separate storage containers (L1, L2), a first and a second storage container, and
that the fermenter (F) is fed with the starting material according to the following steps:
a) feeding of the fermenter (F) with starting material from the second storage container (L2) and filling of a third empty storage container (L3) with technically unusable fermented residual material (R) from the fermenter (F) until the second storage container (L2) is completely emptied,
b) feeding of the fermenter (F) with starting material from the first storage container (L1) and filling of the second storage container (L2) with technically unusable fermented residual material (R) from the fermenter (F) until the first storage container (L1) is emptied, and
c) complete emptying of the second and third storage container (L2, L3), filling of the first and second storage container (L1, L2) with starting material and repetition of the steps a) and b).

8. Method according to Claim 7,
**characterized in that** prior to being channelled to the storage containers (L1, L2), the starting material is pre-treated and converted into a pulpy, pumpable consistency, in which the starting material is fed to the fermenter (F).

9. Method according to Claim 8,
**characterized in that** the pre-treatment comprises cleaning and grinding of the harvested crop plants containing the starting material.

10. Method according to any one of Claims 7 to 9,
**characterized in that** the starting material is stored in a pulpy consistency in the absence of air.

11. Method according to any one of Claims 7 to 10,
**characterized in that** during the storage period of the starting material in a pulpy consistency, the starting material is circulated to prevent sedimentary separation processes occurring.

12. Method according to any one of Claims 9 to 11,
**characterized in that** after the pre-treatment of the starting material and before the starting material is fed into the fermenter (F), the fluid content of the starting material existing in a pulpy consistency is reduced by evaporation and/or by means of mechanical dehydration technology.

13. Use of the fermenter system according to any one of Claims 1 to 6 for the all-year round, continuous production of technically usable fermenter products, in particular of biogas.

14. Use according to Claim 13,
**characterized in that** the technically usable fermenter product is biogas.

## Revendications

1. Système fermenteur pour la fermentation en continu de plantes de culture agricole, notamment de navets ou de betteraves ou de produits intermédiaires obtenus à partir de plantes de culture agricole, désignés en outre comme matériau de sortie, comportant un entrepôt de stockage pour entreposer le matériau de sortie, un dispositif d'alimentation pour garnir un fermenteur (F) avec le matériau de sortie stocké provenant de l'entrepôt de stockage ainsi qu'un entrepôt de rendement dans lequel au moyen d'un dispositif de liaison un matériau résiduel fermenté (R) peut être obtenu à partir du fermenteur (F),
**caractérisée en ce que** l'entrepôt de stockage l'entrepôt de rendement sont conçus sous la forme d'au moins conteneurs de stockage (L1, L2, L3), un premier, deuxième et troisième conteneur de stockage, desquels le premier et le deuxième conteneur de stockage (L1, L2) sont reliés par l'intermédiaire d'un dispositif d'alimentation (Z1, Z2) avec le fermenteur (F), par l'intermédiaire duquel le matériau de sortie entreposé dans les conteneurs de stockage (L1, L2) peut être transféré dans le fermenteur (F),
**en ce que** le deuxième et le troisième conteneur de stockage (L2, L3) sont reliés au fermenteur(F) par l'intermédiaire d'au moins un dispositif de liaison (V2, V3), par l'intermédiaire duquel le matériau résiduel (R) fermenté non utilisable techniquement généré dans le fermenteur (F) peut être évacué,
et le deuxième et le troisième entrepôt de stockage (L2, L3) dispose respectivement d'une conduite de vidange (AB), par l'intermédiaire de laquelle le matériau résiduel (R) fermenté entreposé temporairement dans les conteneurs de stockage respectifs (L2, L3) peut être vidé.

2. Système fermenteur selon la revendication 1,
**caractérise en ce que** un dispositif de commande (S) est prévu, qui commande un flux de matériau constitué par le matériau de sortie ou le matériau résiduel (R) fermenté respectivement entre les conteneurs de stockage (L1, L2, L3) et le fermenteur (F) le long du dispositif d'alimentation (Z1, Z2) et du dispositif de liaison (V2, V3) respectif à partir du premier et du deuxième econteneur de stockage (L1, L2) avec le matériau de sortie ainsi qu'un troisième conteneur de stockage vide (L3) de la manière suivante :
a) garnir le fermenteur (F) avec le matériau de sortie provenant du deuxième conteneur de stockage (L2) et remplir le troisième conteneur de stockage (L3) avec le matériau résiduel fermenté (R) provenant du fermenteur (F) jusqu'à ce que le deuxième conteneur de stockage (L2) soit vidé et le troisième conteneur de stockage (L3) soit rempli au moins partiellement,
b) garnir le fermenteur (F) avec le matériau de sortie provenant du premier conteneur de stockage (L1) et remplir le deuxième conteneur de stockage (L2) avec le matériau résiduel fermenté (R) provenant du fermenteur (F) jusqu'à ce que le premier conteneur de stockage (L1) soit vidé et le deuxième conteneur de stockage (L2) soit rempli au moins partiellement et
c) vider le deuxième et le troisième conteneur de stockage (L2, L3), remplir le premier et le deuxième conteneur de stockage (L1, L2) avec le matériau de sortie et répéter les étapes de flux de matériau a) et b).

3. Système fermenteur selon la revendication 1 ou 2, **caractérisé en ce que** le deuxième et le troisième conteneur de stockage (L2, L3) dispose respectivement d'un volume de stockage, qui est proportionné de telle sorte que tout le matériau de sortie stocké dans le deuxième conteneur de stockage (L2) après la fermentation à l'intérieur du fermenteur (F) remplit au moins partiellement sous la forme de matériau résiduel fermenté (R) le volume de stockage du troisième conteneur de stockage (L3), et **en ce que** le premier et le deuxième conteneur de stockage (L1, L2) dispose respectivement d'un volume de stockage, qui est proportionné de telle sorte que tout le matériau de sortie stocké dans le premier conteneur de stockage (L1) après la fermentation à l'intérieur du fermenteur (F) remplit au moins partiellement sous la forme de matériau résiduel fermenté (R) le volume de stockage du deuxième conteneur de stockage (L2).

4. Système fermenteur selon une des revendications 1 à 3,
**caractérisé en ce que** les conteneurs de stockage (L1, L2, L3) sont constitués d'un matériau résistant à la corrosion.

5. Système fermenteur selon une des revendications 1 à 4,
**caractérisé en ce que** au moins chaque conteneur de stockage (L1, L2), dans lequel le matériau de sortie stocké peut être amené par un prétraitement à une consistance visqueuse, présente un dispositif de mélange pour accompagner les processus de séparation par sédimentation.

6. Système fermenteur selon la revendication 5,
**caractérisée en ce que** le dispositif de mélange comporte un malaxeur monté à l'intérieur du conteneur de stockage (L1, L2) et/ou une pompe de circulation.

7. Procédé de fermentation en continu de plantes de culture agricole, notamment de navets ou de betteraves ou de produits intermédiaires obtenus à partir de plantes de culture agricole, désignés en outre comme matériau de sortie, dans lequel le matériau de sortie est stocké dans au moins un conteneur de stockage pour garnir successivement un fermenteur (F), dans lequel le matériau de sortie est converti sous l'action de produits de fermentation en un matériau résiduel fermenté (R), qui est transféré dans un entrepôt de rendement,
**caractérisé en ce que** le stockage du matériau de sortie a lieu dans au moins deux conteneurs de stockage (L1, L2) séparés,
un premier et un deuxième conteneur de stockage et **en ce que** la garniture du fermenteur (F) avec le matériau de sortie a lieu selon les étapes suivantes :
a) garnir le fermenteur (F) avec le matériau de sortie provenant du deuxième conteneur de stockage (L2) et remplir un troisième conteneur de stockage (L3) avec le matériau résiduel fermenté (R) provenant du fermenteur (F) jusqu'à ce que le deuxième conteneur de stockage (L2) soit complètement vidé,
b) garnir le fermenteur (F) avec le matériau de sortie provenant du premier conteneur de stockage (L1) et remplir le deuxième conteneur de stockage (L2) avec le matériau résiduel fermenté (R) provenant du fermenteur (F) jusqu'à ce que le premier conteneur de stockage (L1) soit vidé et
c) vider complètement le deuxième et le troisième conteneur de stockage (L2, L3), remplir le premier et le deuxième conteneur de stockage (L1, L2) avec le matériau de sortie et répéter les étapes de flux de matériau a) et b).

8. Procédé selon la revendication 7,
**caractérisé en ce que** le matériau de sortie est prétraité avant le placement dans le conteneur de stockage (L1, L2) est transformé en une consistance visqueuse, pouvant être pompée, à laquelle le matériau de sortie est alimenté dans le fermenteur (F).

9. Procédé selon la revendication 8,
**caractérisée en ce que** le prétraitement comprend un nettoyage et un broyage des plantes de culture récoltées, contenant le matériau de sortie.

10. Procédé selon une des revendications 7 à 9,
**caractérisée en ce que** le stockage du matériau de sortie a lieu à la consistance visqueuse sous exclusion d'air.

11. Procédé selon une des revendications 7 à 10,
**caractérisée en ce que** pendant le stockage du matériau de sortie dans la consistance visqueuse une recirculation du matériau de sortie a lieu pour éviter des processus de séparation par sédimentation.

12. Procédé selon une des revendications 9 à 11,
**caractérisé en ce que** après le prétraitement du matériau de sortie et avant l'alimentation du matériau de sortie dans le fermenteur (F) la fraction liquide du matériau de sortie présent à la consistance visqueuse est réduite par évaporation et/ou au moyen d'une technique de déshydratation mécanique.

13. Utilisation du système fermenteur selon une des revendications 1 à 6 pour la production en continu toute l'année de produits de fermentation utilisables techniquement, notamment de bio gaz.

14. Utilisation selon la revendication 13,
**caractérisée en ce que** les produits de fermentation utilisables techniquement sont du bio gaz.
